# EUROPEAN PATENT APPLICATION

(11) **EP 4 585 131 A1**
(43) Date of publication of application: **16.07.2025**
(21) Application number: 23862202.1
(22) Date of filing: 25.08.2023
(51) Int. Cl.: A61B 1/00

(54) **ENDOSCOPE PROTECTING SLEEVE**

(30) Priority: 05.09.2022 HK 32022059549
(71) Applicant: Chan, Siu King, NT Hong Kong (HK)
(72) Inventor: Chan, Siu King, NT Hong Kong (HK)
(74) Representative: Isern Patentes y Marcas S.L.
(86) International application number: PCT/CN2023/115056
(87) International publication number: WO 2024/051506

(57) **Abstract**

The present application provides an endoscope protective sleeve, and relates to the technical field of medical apparatus and instruments. The endoscope protective sleeve comprises a pipe sleeve, one terminal end of the pipe sleeve is provided with a pipe opening, and an interior of the pipe sleeve is a pipe cavity that is communicated with the pipe opening; the pipe cavity internally comprises pipes with a variety of radial dimensions, and the pipes with different radial dimensions are adapted to endoscopes with different radial dimensions, respectively; in the pipes, the radial dimensions of the pipes are successively or gradually decreased from the pipe opening in a direction away from the pipe opening. The endoscope protective sleeve is capable of being adapted to endoscope terminal ends with different radial dimensions through the pipe cavity. The pipe sleeve comprises a pipe frame and a plurality of half-ring sleeves; the pipe frame comprises two fixing rods. After the endoscope terminal ends with corresponding outer diameter dimensions pass through the pipe opening, the endoscope terminal ends can be correspondingly clamped on the pipes with corresponding outer diameter dimensions, such that the endoscope terminal ends are capable of being stably assembled in the pipe sleeve after passing through the pipe opening.

## Description

### Cross-references to Related Applications

The present application claims priority to a short-term patent application No. 32022059549.2 filed to the Intellectual Property Department of the Hong Kong Special Administrative Region of the People's Republic of China on September 05, 2022 and entitled "Endoscope Protective Sleeve", the entire contents of which are incorporated herein by reference.

### Technical Field

The present application relates to the technical field of medical apparatus and instruments, specifically an endoscope protective sleeve.

### Background Art

An endoscope is a medical testing instrument that integrates an image sensor, an optical lens, a light source for illumination, and a mechanical device, etc., which is capable of entering the human body to take images to facilitate diagnosis of diseases. When the endoscope needs to be stored after use, a protective sleeve usually needs to be used to cover an end of the endoscope that is provided with a lens, thereby playing a better protection role on a key part of the endoscope.

Two kinds of protective sleeves have been commonly used for the endoscope at present. One kind of protective sleeves with corresponding inner diameter dimensions have been designed for endoscopes with different outer diameter dimensions. Such kind of protective sleeves have the disadvantage that because each protective sleeve is only adapted to and compatible with one endoscope, the types of the protective sleeves are also increased when the endoscopes have many types.

As a result, another kind of protective sleeves that can be simultaneously adapted to endoscopes with a variety of different dimensions have been developed by some manufacturers. For such kind of protective sleeves, the protective sleeves are provided with a plurality of silicone rubber thin films in length directions of pipe cavities for accommodating the endoscopes, and the silicone rubber thin films are provided with + shaped through grooves. The + shaped through grooves are used as assembly positioning ports of the protective sleeves. When the endoscopes pass through the + shaped through grooves, the silicone rubber thin films play the role of clamping surfaces of the endoscopes, thereby preventing the endoscopes from loosening from the protective sleeves and achieving the effect of protecting the endoscopes.

Such kind of protective sleeves have the following defects during use: when the radial dimensions of the endoscopes are relatively large, the silicone rubber thin films can still clamp the surfaces of the endoscopes to achieve a protection effect; however, when the radial dimensions of the endoscopes are relatively small, the silicone rubber thin films usually cannot clamp the surfaces of the endoscopes well, which will cause loosening of the endoscopes from the protective sleeves, such that the endoscopes are not well protected.

### Summary of the Invention

In view of the shortcomings of the prior art, the present application provides an endoscope protective sleeve, which solves the problems that protective sleeves are incapable of being simultaneously adapted to a variety of different endoscopes and clamping is not tight.

To achieve the above objective, the present application is implemented through the following technical solutions: an endoscope protective sleeve, comprising a pipe sleeve, one terminal end of the pipe sleeve is provided with a pipe opening, and the interior of the pipe sleeve is a pipe cavity that is communicated with the pipe opening;
the pipe cavity internally comprises pipes with a variety of radial dimensions, and the pipes with different radial dimensions are adapted to endoscopes with different radial dimensions, respectively;
in the pipes, the radial dimensions of the pipes are successively or gradually decreased from the pipe opening in a direction away from the pipe opening.

Preferably, the pipe sleeve comprises a pipe frame and a plurality of half-ring sleeves; the pipe frame comprises two fixing rods; each of the half-ring sleeves is spanned between the two fixing rods, with two ends connected to the two fixing rods, respectively.

Preferably, two axis lines of the two fixing rods constitute an axis plane; the plurality of half-ring sleeves are arranged into a first half-ring sleeve set and a second half-ring sleeve set in the form of an array;
the first half-ring sleeve set is distributed on a first side of the axis plane, and the second half-ring sleeve set is distributed on a second side of the axis plane; furthermore, the half-ring sleeves composing the first half-ring sleeve set and the half-ring sleeves composing the second half-ring sleeve set are distributed in a staggered manner on the pipe frame.

Preferably, thicknesses of middle portions of the half-ring sleeves are smaller than those of two terminal ends of the half-ring sleeves; the middle portions of the half-ring sleeves refer to the portions of the half-ring sleeves that are connected between the two terminal ends.

Preferably, a through hole is provided in the middle portion of each of the half-ring sleeves.

Preferably, the other terminal end of the pipe sleeve that is away from the pipe opening is provided with a hanging lug.

Preferably, the pipe sleeve is made of and molded by an elastic material; the elastic material comprises rubber or silicone rubber.

Preferably, the pipe opening is covered with a thin film made of the elastic material, and the thin film is provided with a groove.

Preferably, the groove is arranged to have one of a "+" shaped appearance, a "*" shaped appearance, and a character "Y" shaped appearance.

Preferably, the thin film is further provided with dotted slot holes, and the dotted slot holes are distributed along the groove.

Preferably, the thin film comprises a first state and a second state, and the thin film can be unidirectionally transformed from the first state to the second state; when the thin film is in the first state, an endoscope does not enter the pipe cavity; and when the thin film is in the second state, the endoscope can at least partially enter the pipe cavity.

Preferably, the protective sleeve comprises the following parts by weight of raw materials: 40%-50% of PLA, 20%-30% of peanut protein powder, 10%-5% of lignocellulose, 10%-5% of surface-modified chitin whiskers, 5%-2.5% of silicone rubber, 5%-2.5% of hard clay, 5%-2.5% of a photosensitizer, 2.5%-1% of a toughening agent, 10%-5% of amylose, and 5%-2.5% of dehydroacetic acid.

Working principle: The PLA is polylactic acid, also known as polylactide, which is a polyester polymer obtained by polymerization with lactic acid as a main raw material. In addition to having biodegradability, those prepared from the PLA have good biocompatibility, glossiness, transparency, hand feeling and heat resistance. The peanut protein powder means that peanut oil is produced by the processes of red skin removal, low-temperature cold pressing at 60°C and filtration of selected high-quality peanuts; a product is obtained by crushing, low-temperature extraction using a subcritical biotechnology, and crushing and sterilization of cold-rolled oilseed meal. The lignocellulose is toxic, [P1] tasteless, pollution-free and non-radioactive and has excellent flexibility and dispersivity. The surface-modified chitin whiskers can be dissolved in dimethylacetamide containing 8% lithium chloride or concentrated acid. The silicone rubber refers to rubber with a main chain alternately composed of silicon atoms and oxygen atoms and the silicon atoms usually connected with two organic groups. The silicone rubber is non-toxic, tasteless, odorless and non-adhesive to human tissues, has an anticoagulation effect and little reactivity to body tissues, and is particularly suitable for use as a medical material. The hard clay has plasticity, and wet clay can be molded into various shapes without breaking and can remain unchanged for a long time. Through the photosensitizer, the protective sleeve is free of the phenomena of yellowing and aging after curing. The toughening agent is a substance capable of improving the flexibility of an adhesive film layer of the protective sleeve. The amylose has properties similar to those of fibers. A thin film made of the amylose has good transparency, flexibility, tensile strength and water insolubility, can be applied to the production of sealing materials, packaging materials and water-resistant and pressure-resistant materials, has a certain antibacterial effect under acid or alkali conditions, and especially has a strongest inhibitory effect on mold. According to the endoscope protective sleeve prepared from the above raw materials, the protective sleeve has good degradability, the endoscope protective sleeve can also have certain transparency, toughness, and aging resistance, etc., and the endoscope protective sleeve has a certain antibacterial effect.

The present application provides an endoscope protective sleeve. The present application has the following beneficial effects:

According to the present application, the endoscope protective sleeve is capable of being adapted to endoscope terminal ends with different radial dimensions through the pipe cavity. After the endoscope terminal ends with corresponding outer diameter dimensions pass through the pipe opening, the endoscope terminal ends can be correspondingly clamped on the pipes with corresponding outer diameter dimensions, such that the endoscope terminal ends are capable of being stably assembled in the pipe sleeve after passing through the pipe opening.

### Brief Description of the Drawings

One or more embodiments are exemplarily illustrated through figures in corresponding drawings, and these exemplary illustrations do not constitute limitations to the embodiments. Elements with same reference numerals in the drawings are represented as similar elements. Unless otherwise stated, the figures in the drawings do not constitute scale limitations.
FIG. 1 is a perspective view of an endoscope protective sleeve provided in an embodiment of the present application;
FIG. 2 is a cross-sectional view of an endoscope protective sleeve provided in an embodiment of the present application, showing section conditions in an A-A direction in FIG. 1;
FIG. 3 is a front view of an endoscope protective sleeve provided in an embodiment of the present application, showing conditions observed in a B direction in FIG. 1;
FIG. 4 is a perspective view of another endoscope protective sleeve provided in an embodiment of the present application.

In the drawings: 100, endoscope protective sleeve; 10, pipe sleeve; 11, pipe opening; 12, pipe cavity; 121, pipe; 122, pipe; 123, pipe; 13, half-ring sleeve; 14, fixing rod; X1, axis line; X2, axis line; R1, first half-ring sleeve set; R2, second half-ring sleeve set; 131, middle portion of the half-ring sleeve; 132, terminal end; 133, through hole; 20, hanging lug; 15, thin film; 151, groove; 152, dotted slot hole.

### Detailed Description of Embodiments

To make the objectives, technical solutions and advantages of the embodiments of the present application more clear, the technical solutions in the embodiments of the present application are clearly and completely described below in conjunction with the accompanying drawings in the embodiments of the present application. Obviously, the described embodiments are only some of the embodiments of the present application, rather than all of the embodiments. It should be understood that the specific embodiments described herein are only used to explain the present application and are not used to limit the present application. Based on the embodiments in the present application, all other embodiments obtained by those of ordinary skill in the art without exerting creative labor fall within the scope of protection of the present application.

It should be noted that when an element is described as "fixed to" another element, it can be directly on the other element, or one or more centered elements may exist therebetween. When an element is described as "connected to" another element, it can be directly connected to the other element, or one or more centered elements may exist therebetween. The terms "vertical", "horizontal", "left", "right" and similar expressions used in the specification are only for the purpose of illustration.

### Embodiment 1:

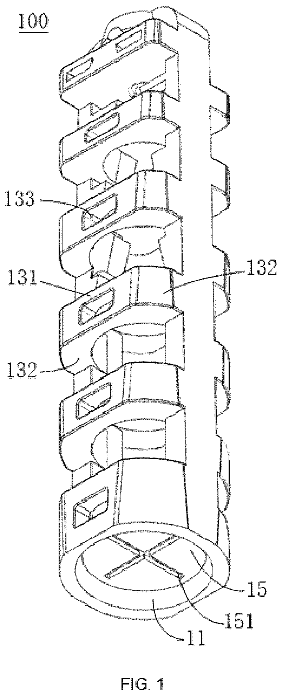

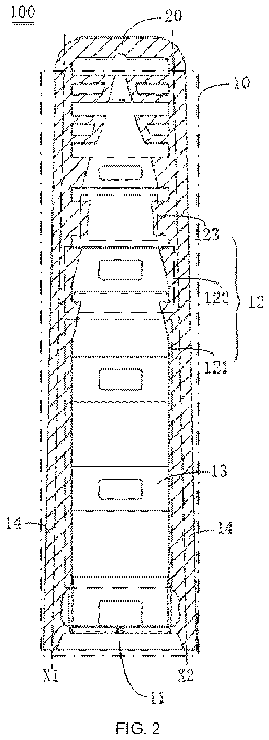

As shown in FIG. 1 and FIG. 2, an endoscope protective sleeve 100 provided in the embodiment of the present application may comprise: a pipe sleeve 10.

One terminal end of the pipe sleeve 10 is provided with a pipe opening 11, and an interior of the pipe sleeve 10 is a pipe cavity 12 that is communicated with the pipe opening 11. The pipe opening 11 is used for allowing a terminal end of an endoscope that is provided with a lens (hereinafter referred to as "endoscope terminal end") to pass through, and the pipe cavity 12 is used for accommodating the endoscope terminal end passing through the pipe opening 11.

The pipe cavity 12 internally comprises pipes (a first pipe 121, a second pipe 122 and a third pipe 123) with a variety of radial dimensions, and the pipes with different radial dimensions are adapted to endoscopes with different radial dimensions, respectively. In the pipes, the radial dimensions of the pipes are successively or gradually decreased from the pipe opening 11 in a direction away from the pipe opening 11.

Specifically, the pipes may comprise the first pipe 121, the second pipe 122 and the third pipe 123, and the radial dimensions of the first pipe 121, the second pipe 122 and the third pipe 123 in the pipe cavity 12 are successively or gradually decreased from one end of the pipe opening 11 in the direction away from the pipe opening 11, and are correspondingly adapted to a large-size endoscope, a medium-size endoscope and a small-size endoscope, respectively. In the embodiment of the present application, the radial dimension of the large-size endoscope may be 13-20 mm, the radial dimension of the medium-size endoscope may be 8-12 mm, and the radial dimension of the small-size endoscope may be 2.5-5 mm.

It should be noted that the so-called "successively" means that for the endoscope protective sleeve 100 in the pipe cavity 12 from the pipe opening 11 in the direction away from the pipe opening 11, the radial dimension of the pipe cavity 12 of the endoscope is a set of numerical values that are gradually decreased from large to small, and the numerical values show step changes from large to small, which is different from the case that the numerical values are continuously decreased in a cone-shaped manner from the pipe opening 11 in the direction away from the pipe opening 11. Certainly, the radial dimension of the pipe cavity 12 of the endoscope may also be continuously decreased in a cone-shaped manner from the pipe opening 11 in the direction away from the pipe opening 11.

Through the above design, the endoscope protective sleeve 100 can enable the pipe cavity 12 of the endoscope to be adapted to endoscope terminal ends with different radial dimensions. After the endoscope terminal ends with corresponding outer diameter dimensions pass through the pipe opening 11, the endoscope terminal ends can be correspondingly clamped on the pipes with corresponding outer diameter dimensions, such that the endoscope terminal ends are capable of being stably assembled in the pipe sleeve 12 [P2]after passing through the pipe opening 11, thereby achieving reliable covering and protecting effects of the endoscope protective sleeve 100 on the endoscope terminal ends in some occasions (for example, transportation, storage, and disinfection).

In the embodiment of the present application, the pipe sleeve 10 or the endoscope protective sleeve 100 can be made of and molded by an elastic material as a whole, and the elastic material can be, but is not limited to, rubber or silicone rubber that is commonly used at present.

In some embodiments, to prevent the endoscope from falling out of the endoscope protective sleeve 100 due to looseness after the endoscope terminal end is accommodated in the endoscope protective sleeve 100, the radial dimensions of the various pipes of the endoscope protective sleeve 100 can be designed to be slightly smaller than the radial dimensions of corresponding endoscope terminal ends to which the various pipes are assembled.

After the endoscope terminal end passes through the pipe opening 11, since the radial dimension of the pipe cavity of the endoscope protective sleeve 100 is smaller than that of the endoscope terminal end, the endoscope terminal end can squeeze inner walls of the pipes to make the pipes squeezed and deformed, and accordingly, the pipes have an elastic covering force of a certain size, such that the endoscope terminal end can be stably and firmly covered in the pipe cavity 12.

As shown in FIG. 2, in some embodiments, the pipe sleeve comprises a pipe frame and a plurality of half-ring sleeves 13. The pipe frame comprises two fixing rods 14; each of the half-ring sleeves 13 is spanned between the two fixing rods 14, with two ends connected to the two fixing rods 14, respectively. Thus, the two fixing rods 14 and the plurality of half-ring sleeves 13 are connected to form an integral base frame of the endoscope protective sleeve 100.

Specifically, the two fixing rods 14 can be parallel to each other, and can also approach to intersection at a preset angle. Respective axis lines (X1, X2) of the two fixing rods 14 constitute a plane. For convenience of description, the plane is called an axis plane.

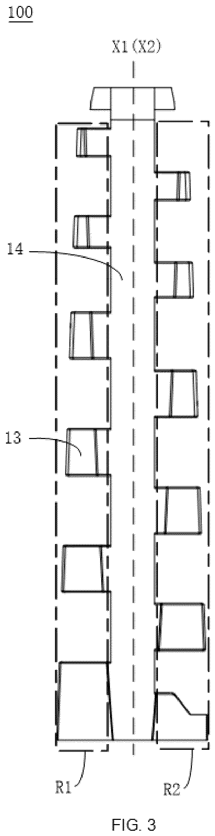

As shown in FIG. 3, on the pipe sleeve 10, with the axis plane constituted by the axis lines (X1, X2) as a boundary, the plurality of half-ring sleeves 13 can be divided into a first half-ring sleeve set R1 and a second half-ring sleeve set R2. The plurality of half-ring sleeves 13 are arranged into the first half-ring sleeve set R1 in the form of an array and then distributed on a first side of the axis plane, the plurality of half-ring sleeves 13 are arranged into the second half-ring sleeve set R2 in the form of an array and then distributed on a second side of the axis plane, and the half-ring sleeves 13 of the first half-ring sleeve set R1 and the half-ring sleeves 13 of the second half-ring sleeve set R2 are distributed in a staggered manner on the pipe frame.

Certainly, the half-ring sleeves 13 of the first half-ring sleeve set R1 and the half-ring sleeves 13 of the second half-ring sleeve set R2 can also be arranged oppositely on the pipe frame, that is, two terminal ends of the half-ring sleeves 13 on the first side (the first half-ring sleeve set R1) and two terminal ends of the half-ring sleeves 13 on the second side (second half-ring sleeve set R2) are connected at same positions on the two fixing rods 14, and the half-ring sleeves 13 on the first side (the first half-ring sleeve set R1) and the half-ring sleeves 13 on the second side (second half-ring sleeve set R2) form a ring shape.

Compared with the method that the half-ring sleeves 13 on the two sides are arranged oppositely, the method that the half-ring sleeves 13 on the two sides are distributed in a staggered manner can make a covering force distributed to the endoscope terminal end in a length direction of the endoscope, the covering force of the endoscope protective sleeve 100 for the endoscope terminal end is distributed more dispersively, and the situation can be avoided that the endoscope terminal end cannot smoothly enter the pipe cavity 12 or be smoothly removed from the pipe cavity 12 due to a too large covering force.

As shown in FIG. 1, in some embodiments, thicknesses of middle portions 131 of the half-ring sleeves are smaller than those of terminal ends 132 of the half-ring sleeves. It should be noted that the middle portions 121[P3] of the half-ring sleeves refer to the portions on the half-ring sleeves 13 that are connected between the two terminal ends 132 of the half-ring sleeves, and the terminal ends 132 of the half-ring sleeves refer to the two terminal ends on the half-ring sleeves 13 that are used to be connected to the fixing rods 14; the thickness refers to a radial dimension parameter of the endoscope protective sleeve 100.

Such design method of the half-ring sleeves 13 has the advantages that the half-ring sleeves 13 are connected to the fixing rods 14 at the terminal ends 132 with a larger thickness, which can ensure the connection strength between the half-ring sleeves 13 and the fixing rods 14. In the process of the endoscope terminal end entering the pipe cavity of the endoscope protective sleeve 100, the thinner middle portions 131 of the half-ring sleeves are conducive to better deformation, such that the endoscope terminal end can more easily enter the pipe cavity 12 of the endoscope protective sleeve 100.

Continuously referring to FIG. 1, in some embodiments, a through hole 133 may be provided in the middle portion of each of the half-ring sleeves 13. The design of the through hole 133 is conducive to further improving the elastic deformation capacity of the middle portions of the half-ring sleeves 131, such that after the endoscope terminal end enters the pipe cavity 12, each of the half-ring sleeves 13 can cover the surface of the endoscope with a more appropriate elastic covering force serving as a pre-tightening force to restrict the endoscope from loosening from the endoscope protective sleeve 120.[P4]

The pipe opening 11 is provided with a thin film 15 made of and molded by the above-mentioned elastic material, and the thin film 15 covers the pipe opening 11. The thin film 15 is provided with a groove 151, and a thickness of the groove 151 on the thin film 15 is smaller than that of another portion except for the groove 151 on the thin film 15, facilitating that when the endoscope protective sleeve 100 is used to cover the endoscope terminal end, the thin film 15 can be easily pierced by the endoscope terminal end due to the thinner groove 151, and thus the endoscope terminal end smoothly enters the pipe cavity 12.

In the embodiment of the present application, the groove 151 can be arranged to have a "+" shaped appearance, or a "*" shaped appearance, or a "Y" shaped appearance, and the appearance can be designed in an appropriate shape according to actual needs. In the embodiment of the present application, the appearance of the groove 151 is not specifically limited.

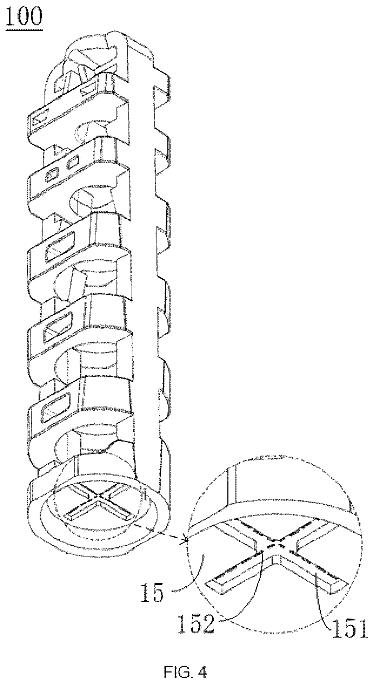

As shown in FIG. 4, in some embodiments, to facilitate the thin film 15 to be better pierced, a plurality of slot holes 152 that are communicated with the pipe cavity 12 may also be provided at the bottom of the groove 151 on the thin film 15 in an extension direction of the groove 151, and the plurality of slot holes 152 are distributed in a dotted line shape in the groove 151. For convenience of description, the plurality of slot holes 152 distributed in a dotted line shape are referred to as "dotted slot holes 152" below.

When the endoscope terminal end is used to pierce the thin film, since the thin film is designed with the dotted slot holes 152, the thin film is directionally torn into an opening in the direction of the dotted slot holes 152, and the endoscope terminal end extends into the pipe cavity 12 through the opening. Thus, it can be seen that the design of the dotted slot holes 152 can effectively reduce burrs generated on an edge of the opening after the thin film 15 is torn, and can improve covering and positioning effects of the thin film 15 on the endoscope terminal end.

It should be noted that structures of the groove 151 and the dotted slot holes 152 on the thin film 15 also have a certain marking function while facilitating the endoscope terminal end to pierce through the thin film 15, that is, a user can judge whether the endoscope protective sleeve 100 has been used according to the states of the groove 151 and the dotted slot holes 152, thereby preventing the user from using the endoscope protective sleeve 100 for several times. For example, when the groove 151 and the dotted slot holes 152 are pierced and the thin film 15 is pierced to form an opening, at this time, the endoscope protective sleeve 100 is obviously in a use state or a state that has been used, and the user can replace another endoscope protective sleeve 100 for use.

In some embodiments, the thin film 15 comprises a first state and a second state, and the thin film 15 can be unidirectionally transformed from the first state to the second state; when the thin film 15 is in the first state, an endoscope does not enter the pipe cavity 12; and when the thin film 15 is in the second state, the endoscope can at least partially enter the pipe cavity 12. It can be understood that the first state of the thin film 15 refers to the state in which the groove 151 and/or the dotted slot holes 152 located thereon are not pierced, and at this time, the endoscope does not enter the pipe cavity 12. The second state of the thin film 15 refers to the state in which the groove 151 and/or the dotted slot holes 152 located thereon are pierced, at this time, the thin film 15 is pierced into an opening, and the endoscope terminal end can extend into the pipe cavity 12 through the opening.

As shown in FIG. 2, in some embodiments, the other terminal end of the pipe sleeve 10 that is away from the pipe opening 11 can be provided with a hanging lug 20 according to actual needs, and the hanging lug 20 is connected between terminal ends of the two fixing rods 14. After an endoscope is assembled in the endoscope protective sleeve 100, the endoscope protective sleeve 100 accommodating the endoscope can be hung at a designated position through the hanging lug 20, thereby reducing occupied space.

### Embodiment 2:

The embodiment of the present application provides an endoscope protective sleeve, comprising the following parts by weight of raw materials: 40%-50% of PLA, 20%-30% of peanut protein powder, 10%-5% of lignocellulose, 10%-5% of surface-modified chitin whiskers, 5%-2.5% of silicone rubber, 5%-2.5% of hard clay, 5%-2.5% of a photosensitizer, 2.5%-1% of a toughening agent, 10%-5% of amylose, and 5%-2.5% of dehydroacetic acid.

### Embodiment 3:

The embodiment of the present application provides an endoscope protective sleeve, comprising the following parts by weight of raw materials: 40% of PLA, 20% of peanut protein powder, 5% of lignocellulose, 5% of surface-modified chitin whiskers, 5% of silicone rubber, 5% of hard clay, 5% of a photosensitizer, 2.5% of a toughening agent, 10% of amylose, and 2.5% of dehydroacetic acid.

Although the embodiments of the present application have been shown and described, for those of ordinary skill in the art, it can be understood that various changes, modifications, substitutions and alternations can be made to these embodiments without departing from the principles and spirit of the present application. The scope of the present application is defined by the appended claims and equivalents thereof.

## Claims

1. An endoscope protective sleeve, comprising a pipe sleeve (10), wherein one terminal end of the pipe sleeve (10) is provided with a pipe opening (11), and an interior of the pipe sleeve (10) is a pipe cavity (12) that is communicated with the pipe opening (11);
the pipe cavity (12) internally comprises pipes (121, 122, 123) with a variety of radial dimensions, and the pipes with different radial dimensions are adapted to endoscopes with different radial dimensions, respectively;
in the pipes, the radial dimensions of the pipes are successively or gradually decreased from the pipe opening (11) in a direction away from the pipe opening (11).

2. The endoscope protective sleeve according to Claim 1, wherein the pipe sleeve (10) comprises a pipe frame and a plurality of half-ring sleeves (13); the pipe frame comprises two fixing rods (14); each of the half-ring sleeves (13) is spanned between the two fixing rods (14), with two ends connected to the two fixing rods (14), respectively.

3. The endoscope protective sleeve according to Claim 1, wherein two axis lines (X1, X2) of the two fixing rods (14) constitute an axis plane; the plurality of half-ring sleeves (13) are arranged into a first half-ring sleeve set (R1) and a second half-ring sleeve set (R2) in a form of an array;
the first half-ring sleeve set (R1) is distributed on a first side of the axis plane, and the second half-ring sleeve set (R2) is distributed on a second side of the axis plane; furthermore, the half-ring sleeves (13) composing the first half-ring sleeve set (R1) and the half-ring sleeves (13) composing the second half-ring sleeve set (R2) are distributed in a staggered manner on the pipe frame.

4. The endoscope protective sleeve according to Claim 1, wherein thicknesses of middle portions (131) of the half-ring sleeves are smaller than those of two terminal ends (132) of the half-ring sleeves (13); the middle portions (131) of the half-ring sleeves refer to portions on the half-ring sleeves (13) that are connected between the two terminal ends (132).

5. The endoscope protective sleeve according to Claim 1, wherein a through hole (133) is provided in the middle portion (131) of each of the half-ring sleeves (13).

6. The endoscope protective sleeve according to Claim 1, wherein the other terminal end of the pipe sleeve (10) that is away from the pipe opening (11) is provided with a hanging lug (20).

7. The endoscope protective sleeve according to Claims 1 to 6, wherein the pipe sleeve (10) is made of and molded by an elastic material; the elastic material comprises rubber or silicone rubber.

8. The endoscope protective sleeve according to Claim 7, wherein the pipe opening (11) is covered with a thin film (15) made of the elastic material, and the thin film (15) is provided with a groove (151).

9. The endoscope protective sleeve according to Claim 8, wherein the groove (151) is arranged to have one of a "+" shaped appearance, a "*" shaped appearance, and a character "Y" shaped appearance.

10. The endoscope protective sleeve according to Claim 9, wherein the thin film (15) is further provided with dotted slot holes (152), and the dotted slot holes (152) are distributed along the groove (151).

11. The endoscope protective sleeve according to Claim 8, wherein
the thin film comprises a first state and a second state, and the thin film can be unidirectionally transformed from the first state to the second state; when the thin film is in the first state, an endoscope does not enter the pipe cavity; when the thin film is in the second state, the endoscope can at least partially enter the pipe cavity.

12. The endoscope protective sleeve according to Claim 1, wherein the endoscope protective sleeve comprises the following parts by weight of raw materials: 40%-50% of PLA, 20%-30% of peanut protein powder, 10%-5% of lignocellulose, 10%-5% of surface-modified chitin whiskers, 5%-2.5% of silicone rubber, 5%-2.5% of hard clay, 5%-2.5% of a photosensitizer, 2.5%-1% of a toughening agent, 10%-5% of amylose, and 5%-2.5% of dehydroacetic acid.
